# EUROPÄISCHE PATENTSCHRIFT

(11) **EP 0 711 529 B1**
(45) Veröffentlichungstag und Bekanntmachung des Hinweises auf die Patenterteilung: **27.01.1999**
(21) Anmeldenummer: 95117265.9
(22) Anmeldetag: 02.11.1995
(51) Int. Cl.: A61B 1/12, A61B 19/00

(54) **Verfahren zum Prüfen und Reinigen von Instrumenten für die minimal invasive Chirurgie oder minimal invasive Untersuchung von Körperhöhlen**
Method for examination and cleaning of minimal invasive instruments provided for surgery and exploration of body cavities
Procédé d'examination et de nettoyage d'instruments minimal-invasifs destinés à la chirurgie et à l'exploration de cavités d'un corps

(30) Priorität: 11.11.1994 DE 4440363
(43) Veröffentlichungstag der Anmeldung: 15.05.1996
(73) Patentinhaber: NETZSCH NEWAMATIC GmbH, 84478 Waldkraiburg (DE)
(72) Erfinder: Pfeifer, Egbert, D-84478 Waldkraiburg (DE)
(74) Vertreter: Goetz, Rupert, Dipl.-Ing.

(56) Entgegenhaltungen:
- EP-A- 0 248 632
- CH-A- 679 447
- DE-A- 3 416 743
- DE-A- 3 638 804
- DE-A- 3 918 432
- FR-A- 2 713 111

## Beschreibung

Die Erfindung betrifft ein Verfahren zum Prüfen und Reinigen von Instrumenten für die minimal invasive Chirurgie oder minimal invasive Untersuchung von Körperhöhlen.

Die Einführung der minimal invasiven Chirurgie (MIC) und minimal invasiver Verfahren für die Untersuchung von Körperhöhlen mit speziell für diese Anwendungsbereiche entwickelten chirurgischen bzw. diagnostischen Instrumenten hat die Untersuchungs- und Operationspraxis in wichtigen Bereichen revolutioniert. Diese Instrumente werden durch natürliche oder operativ geschaffene Öffnungen geringen Durchmessers in Körperhöhlungen, wie Darm oder Bauchraum, zum Ort ihres Einsatzes geführt, so daß Untersuchungen und kleinere chirurgische Eingriffe mit wesentlich geringeren Belastungen für den Patienten, beispielsweise ohne Vollnarkose, möglich sind.

Die Klasse der Instrumente für die minimal invasive Chirurgie (MIC) und die minimal invasive Untersuchung von Körperhöhlen umfaßt nicht flexible Schaftinstrumente aus Edelstahl wie auch flexible Instrumente, welche üblicherweise aus Aluminium und hochelastischen Kunststoffmaterialien gefertigt werden.

Nicht flexible Instrumente werden für die minimal invasive Chirurgie eingesetzt und entsprechend als MIC-Instrumente bezeichnet. Sie weisen innerhalb eines langgezogenen Schaftes einen einzelnen Kanal mit einem geringen Durchmesser auf und ermöglichen mit an den Instrumenten vorgesehenen Vorrichtungen, die mittels eines innerhalb des Kanales verlaufenden Kolbens manipuliert werden können, operative Eingriffe, wie beispielsweise das Entfernen der Gallenblase.

Flexible Instrumente, sogenannte Endoskope, werden insbesondere für diagnostische Zwecke eingesetzt und weisen in ihrem Inneren eine Vielzahl von Kanälen und Kapillaren auf.

Eine Verwendung von chirurgischen wie diagnostischen Einweg-Instrumenten soll aus wirtschaftlichen wie ökologischen Gründen möglichst vermieden werden. Deshalb ist es erforderlich, Aufbereitungsverfahren für solche Instrumente zu entwickeln, die eine effektive, den hohen klinischen Anforderungen genügende Reinigung, Desinfektion und Sterilisierung sicherstellen. Während äußere Flächen sowie Gelenke solcher Instrumente von festsitzenden Verunreinigungen durch Ultraschallbehandlung einfach und wirksam gereinigt werden können, ist das Durchspülen und anschließende Reinigen und Sterilisieren eines oder mehrerer Kanäle in röhrenförmigen Bauteilen ein hinsichtlich der Einhaltung strenger Hygienevorschriften kritischer Aufbereitungsschritt.

Für flexible Endoskope, welche mehrere Kanäle insbesondere im Inneren der flexiblen Bereiche aufweisen und aus diesem Grunde entsprechende Probleme bei einer Reinigung und Sterilisierung aufwerfen, wurden Verfahren und Vorrichtungen entwickelt, die eine hohen hygienischen Anforderungen entsprechende Reinigung, Desinfektion und Sterilisierung ermöglichen sollen. In der Zeitschrift "Krankenhaus Technik", Ausgabe Mai 1991, S. 32 bis 39, wird ein Verfahren zum Prüfen, Reinigen und Desinfizieren von Endoskopen beschrieben, wobei die Kanäle der Instrumente an eine Fluidquelle für eine chemothermische Aufbereitung, eine Reinigung unter Verwendung von konzentrierten Wasch- und Desinfektionslösungen unter erhöhter Temperatur mit anschließender steriler Nachspülung, angeschlossen werden. Da die Endoskopkanäle innerhalb eines geschlossenen Systems, das auch die Fluidquelle umfaßt, durchspült werden, können die Kanäle innerhalb des Verfahrensablaufs ohne größeren Aufwand auch auf Dichtigkeit geprüft werden.

Eine Durchlässigkeitsprüfung sämtlicher Kanäle innerhalb eines Endoskops ist jedoch im Rahmen dieses Verfahrens nicht möglich. Aus diesem Grunde kann nach Abschluß eines solchen Verfahrensablaufs nicht mit Sicherheit ausgeschlossen werden, daß ein Teil der Kanäle während des gesamten Verfahrensablaufs verstopft und damit einer Reinigung und Sterilisation nicht zugänglich war.

In der DE 39 18 432 C2 ist ein Verfahren zum Desinfizieren von Endoskopen offenbart, bei welchem ein Kanal eines solchen Instruments an eine Fluidquelle angeschlossen wird, der Kanal auf Durchlässigkeit geprüft wird, indem ihm Fluid aus der Fluidquelle zugeführt wird, bei zu geringer oder fehlender Durchströmung das betreffende Instrument als nicht ausreichend durchspülbar erkannt und zur Aussonderung registriert und bei ausreichender Durchströmung das betreffende Instrument als durchspülbar erkannt und mit Desinfektions- und/oder Reinigungsmitteln nachgespült wird. Die Wiederherstellung der Durchlässigkeit des Kanals bei nicht ausreichender Durchspülbarkeit wird hierbei manuell vorgenommen.

Aus der GB 2 275 341 A ist ein Verfahren zum Überprüfen von Hohlräumen oder Kanälen in Turbinenschaufeln bekannt, bei dem die Durchströmungsrate eines Fluids durch diese Hohlräume oder Kanäle ermittelt wird und die erhaltenen Daten mit bekannten Werten verglichen werden. Das Ergebnis dieses Vergleichs wird für Aussagen herangezogen, ob sich Festmaterial aus dem Herstellungsverfahren in den Hohlräumen oder Kanäle befindet und in welchem Ausmaß gegebenenfalls eine Verlegung vorliegt.

In der DE 36 38 804 A1 wird ein Reinigungsgerät, insbesondere für die vollautomatische Reinigung und Desinfektion von Endoskopen und Herzkathetern mit einer desinfektionsmittelhaltigen Reinigungsflüssigkeit beschrieben, das aus einer an eine Wasserzuführung anschließbaren Dosiervorrichtung, mindestens einem Konzentratbehälter, gegebenenfalls mindestens einem Druckminderventil und einem Anschlußteil besteht, wobei diese Teile des Gerätes durch Leitungen miteinander verbunden sind, und das dadurch gekennzeichnet ist, daß es mindestens eine mit der Wasserzufuhr und der Dosiervorrichtung verbundene Zeituhr, mindestens eine mit der Wasserzufuhr und der Dosiervorrichtung verbundene Wasseruhr sowie mindestens eine mit der Wasseruhr verbundene Sensorautomatik aufweist. Die Wasseruhr dient zur Überprüfung des Wasserverbrauchs bei unterschiedlichen Durchmessern der Geräte oder Schläuche und dient ferner in Zusammenwirken mit der Sensorautomatik zum Abschalten des Geräts und Unterbrechen des Reinigungsvorganges insbesondere in Fällen von Verstopfungen im Gerät oder in den Schläuchen. Die Sensorautomatik ist so eingestellt, daß sie auf eine Verminderung oder Verhinderung der Durchflußmenge bzw. -zeit in Abweichung von den Normalwerten reagiert und mittels elektrischem oder mechanischem Kontakt die Wasseruhr betätigt und dadurch die Wasserzuführung und auch die Zuführung des Konzentrates abschaltet.

In Krankenhäusern sind gegenwärtig Instrumente für die minimal invasive Chirurgie wie für minimal invasive Verfahren zur Untersuchung von Körperhöhlen mit unterschiedlichen Abmessungen in verschiedenen Anwendungsbereichen im Einsatz. Diese Instrumente unterscheiden sich insbesondere in der Anzahl und den Durchmessern der in ihnen vorgesehenen Kanäle. Um bei einer Reinigung der Instrumente die strengen Hygienevorschriften einhalten und insbesondere eine vollständige Durchlässigkeit sämtlicher Kanäle und Kapillaren als Voraussetzung für eine vollständige Reinigung sicherstellen zu können, wäre es wünschenswert, die Prüfungsparameter und gegebenenfalls auch die Reinigungsbedingungen an den einzelnen Gerätetyp anzupassen.

Es besteht somit ein Bedarf an einem Prüfungs-, Reinigungs- und Desinfektionsverfahren für Instrumente für die minimal invasive Chirurgie und minimal invasive Untersuchung von Körperhöhlen verschiedener Gerätetypen, welches ermöglicht, die Durchlässigkeit sämtlicher Kanäle und Kapillaren im Inneren eines jeden einzelnen Instruments und, in Folge, eine vollständige Reinigung und Desinfektion nach strengen Hygienevorschriften sicherzustellen.

Darüberhinaus ist es wünschenswert, ein derartiges Verfahren so durchzuführen, daß für jeden Instrumententyp individuell die Prüf- und Reinigungsbedingungen vollautomatisch, d.h. ohne Einsatz von Personal, angepaßt werden können.

Die Erfindung hat zur Aufgabe, ein Verfahren zum Prüfen, Reinigen und Desinfizieren von Instrumenten für die minimal invasive Chirurgie und minimal invasive Untersuchung von Körperhöhlen zur Verfügung zu stellen, durch die verunreinigte Instrumente unterschiedlicher Bauart hinsichtlich einer vollständigen Durchlässigkeit sämtlicher, in ihnen verlaufender Kanäle und Kapillaren geprüft und nach strengen Hygienevorschriften gereinigt und desinfiziert werden können.

Es ist eine weitere Aufgabe der Erfindung, ein solches Verfahren so bereitzustellen, daß die Prüf- und Reinigungsbedingungen für jeden Instrumententyp individuell und vollautomatisch, d.h. ohne Einsatz von Personal, angepaßt werden können.

Diese Aufgaben sind, soweit sie ein Verfahren betreffen, mit den Merkmalen des Anspruchs 1 gelöst. Vorteilhafte Weiterbildungen sind Gegenstand der Unteransprüche.

Die Erfindung betrifft ein
Verfahren zum Prüfen und Reinigen von einem oder mehreren Instrument(en) für die minimal invasive Chirurgie oder minimal invasive Untersuchung von Körperhöhlen mit mindestens je einem durchgehenden Kanal, in dem
(a) der Kanal des bzw. jedes Instruments an eine Fluidquelle angeschlossen wird;
(b) der Kanal auf Durchlässigkeit geprüft wird;
(c) bei zu geringer oder fehlender Durchströmung das betreffende Instrument als nicht ausreichend durchspülbar erkannt und zur Aussonderung registriert wird und
(d) bei ausreichender Durchströmung das betreffende Instrument als durchspülbar erkannt, und mit Desinfektions- und/oder Reinigungsmitteln nachgespült wird,
   dadurch gekennzeichnet, daß
   der Kanal auf Durchlässigkeit geprüft wird, indem ihm Fluid aus der Fluidquelle zugeführt, eine Menge des in einer Zeiteinheit den Kanal durchströmenden Fluids bestimmt und das bestimmte Fluidvolumen mit gerätespezifisch vorgegebenen Daten verglichen wird und daß anhand des Ergebnisses der Durchlässigkeitsprüfung das bzw. die Instrument(e) hinsichtlich seines bzw. ihres Gerätetyps identifiziert werden und abhängig davon Bedingungen für das Nachspülen mit Desinfektions- und/oder Reinigungsmitteln festgelegt werden.

Der Erfindung liegt die Erkenntnis zugrunde, daß Instrumente für die minimal invasive Chirurgie und minimal invasive Untersuchung von Körperhöhlen anhand eines in einer Zeiteinheit sämtliche Kanäle und Kapillaren eines Instrumentes durchströmenden Fluidvolumens identifizierbar sind. Das in einer Zeiteinheit durch das Kanal- und Kapillarsystem eines solchen Instrumentes hindurchströmende Fluidvolumen wird von der Anzahl und dem Innendurchmesser dieser Kanäle und Kapillaren unmittelbar bestimmt und ist somit für den Instrumententyp charakteristisch, so daß bei Kenntnis dieser Kenngröße der Gerätetyp unschwer durch Vergleich mit einem vorab an dem Gerätetyp bestimmten Standardwert ermittelt werden kann.

In einer bevorzugten Ausführungsform des erfindungsgemäßen Verfahrens werden die zu reinigenden Instrumente einzeln auf Durchlässigkeit überprüft. Dies bietet sich insbesondere dann an, wenn Instrumente unterschiedlicher Instrumententypen in einem gemeinsamen Verfahrensablauf gereinigt werden sollen.

Werden Instrumente gleichen Typs verwendet, besteht jedoch auch die Möglichkeit, mittels einer gemeinsamen, gleichzeitigen Überprüfung eine ausreichende Durchspülbarkeit sämtlicher Instrumente zu bestätigen, wird das rechnerisch bestimmte erforderliche Gesamtdurchflußvolumen beim Bestimmen des Durchflußvolumens bei gleichzeitiger Durchströmung erzielt.

Die Durchlässigkeit kann so geprüft werden, daß dem bzw. jedem Kanal eines Instrumentes Fluid aus einer Fluidquelle zugeführt, das Volumen des in einer Zeiteinheit den Kanal durchströmenden Fluids gemessen und das gemessene Fluidvolumen mit gerätespezifisch vorgegebenen Daten verglichen wird.

Die Durchflußmenge wird in einer bevorzugten Ausführungsform des erfindungsgemäßen Verfahrens auslaßseitig, also nach Durchströmen der Kanäle, bestimmt. Es ist in einer weiteren Ausführungsvariante des Verfahrens entsprechend der Erfindung jedoch auch möglich, die einen Kanal durchströmende Fluidmenge einlaßseitig zu bestimmen.

In einer alternativen Ausführungsform der Erfindung wird der mindestens eine Kanal eines oder mehrerer Instrumente auf Durchlässigkeit geprüft, indem dieser Kanal mit einem Druck durch Zuführen von Fluid aus einer unter Druck stehenden Fluidquelle beaufschlagt wird, der Druckabfall mit der Zeit infolge eines Fluiddurchstroms durch den Kanal gemessen und der ermittelte Druckabfall mit gerätespezifisch vorgegebenen Daten verglichen wird. Bei Verwendung von Fluiden mit vernachlässigbarer Kompressibilität ist der Druckabfall je Zeiteinheit direkt dem aus dem Fluidreservoir austretenden Flüssigkeitsvolumen proportional und kann somit zu einer indirekten Ermittlung des in einer Zeiteinheit die Kanäle und Kapillaren durchströmenden Fluidvolumens herangezogen werden.

Ergibt der Vergleich des Prüfergebnisses mit gerätespezifisch vorgegebenen Daten eine Übereinstimmung mit für einen speziellen Instrumententyp bekannten oder vorab ermittelten Daten, so wird das der Prüfung unterworfene Instrument als ein Instrument dieses Instrumententyps identifiziert und als durchspülbar erkannt. Aufgrund dieses Ergebnisses wird das betreffende Instrument nachfolgend mit Desinfektions- und/oder Reinigungsmitteln nachgespült.

Bei dem erfindungsgemäßen Verfahren wird in Abhängigkeit vom Ergebnis der Durchlässigkeitsprüfung, d.h. nach Identifizierung der zu reinigenden Instrumente, auch automatisch ein speziell für diesen Gerätetyp vorgesehenes Reinigungs- und gegebenenfalls auch Desinfektionsprogramm gewählt.

Fällt eine Aufbereitung von nicht flexiblen MIC-Instrumenten und Endoskopen gleichermaßen an, so werden die Desinfektions- und Reinigungsbedingungen bevorzugt an den sehr unterschiedlich komplexen inneren Aufbau sowie die unterschiedlichen Materialien der zu reinigenden Instrumente angepaßt. Dies betrifft beispielsweise die Konzentration an Desinfektions- und/ oder Reinigungsmittel in der Spüllauge, die Spüldauer oder das einzusetzende Volumen an Spüllauge, die Temperatur während des Desinfektions- und Reinigungsverfahrens wie auch den höchstzulässigen Druck, mit dem ein Instrument während des erfindungsgemäßen Verfahrens belastet werden darf.

Das erfindungsgemäße Verfahren bietet darüberhinaus die Möglichkeit, in Abhängigkeit vom Ergebnis der Durchlässigkeitsprüfung die Instrumente einzeln oder in Gruppen zusammengefaßt mit Desinfektions- und/oder Reinigungsmitteln nachzuspülen. Klassifiziert man insbesondere nicht flexible MIC-Instrumente nach dem Durchmesser ihres inneren Kanals, können die Instrumente in solche mit relativ engen Kanaldurchmessern, beispielsweise 5 mm, und solche mit relativ großen Kanaldurchmessern, beispielsweise 10 mm, eingeteilt werden. Bei gleichzeitigem Nachspülen von Instrumenten mit unterschiedlichen Durchmessern könnte die Gefahr bestehen, daß bei gegebener Pumpenleistung einzelne Instrumente oder einzelne Instrumententypen ungleichmäßig, d.h. nicht mit dem vorher definierten Durchströmdruck oder Durchströmvolumen, durchspült werden.

Für ein gemeinsames Durchspülen eignen sich insbesondere Instrumente mit engen Kanaldurchmessern. Bei gleichzeitigem Durchspülen mehrerer Instrumente mit relativ großen Kanaldurchmessern kann unter Umständen die Leistung der verwendeten Pumpe nicht mehr genügen, um den vorher definierten Durchströmdruck oder das vorher festgelegte Durchströmvolumen zu erreichen, so daß solche Instrumente bevorzugt einzeln oder allenfalls in kleinen Gruppen nachgespült werden. Durch diese Maßnahmen kann sichergestellt werden, daß sämtliche Instrumente unter definierten Bedingungen nachgespült werden.

Der Verfahrensschritt der Durchströmbarkeitsmessung bewirkt zugleich eine erste Vorreinigung. Für die sich an diesen Schritt anschließenden Desinfektions- und Reinigungsschritte sind keine größeren Umstellungen, sondern allenfalls ein Wechsel der Fluidquelle bzw. ein Zudosieren von Desinfektions- und/oder Reinigungsmittel zu dem im Reservoir vorhandenen Fluid erforderlich, falls für die Prüfung ein anderes Fluid als für die Reinigung und Desinfektion verwendet wird.

Im Falle fehlender oder unzureichender Durchströmung können automatisch zusätzliche Maßnahmen ergriffen werden, um zu einer vollständigen Durchspülbarkeit zu gelangen. Hierzu kann ein pneumatischer oder hydraulischer Druckstoß eingesetzt werden. Alternativ ist es möglich, bei Verwendung einer Umwälzpumpe für die Fluidzuführung diese mit erhöhter Drehzahl und dementsprechend höherem Druck zu fahren; hierzu können auch polumschaltbare Kreisel- oder Verdrängerpumpen eingesetzt werden. Um höhere Drücke zu erzeugen, können auch zwei Pumpen hintereinandergeschaltet werden.

Wird ein ordnungsgemäßer Durchfluß festgestellt, so wird der Prüfzyklus, wie beschrieben, fortgesetzt. Wird jedoch weiterhin registriert, daß der Kanal des fraglichen Instruments verstopft ist, wird dieses Gerät bzw. dessen Position im Mikroprozessor automatisch registriert und dieses Gerät kann dann manuell entnommen, überprüft und neu eingesetzt werden.

Im Anschluß an dieses Prüfverfahren werden die unbeanstandeten Instrumente aus einem mit Reinigungs- und Desinfektionsmittel(n) beschickten Reservoir nachgespült. Dabei kann die eingesetzte Spülflüssigkeit gesammelt und erneut dem Reservoir zugeführt werden. Das Fluid aus der Fluidquelle wird dabei üblicherweise von einer Umwälzpumpe gefördert.

Hartnäckige Verschmutzungen an Oberflächen und Gelenken der Instrumente können durch zusätzliche Ultraschallbehandlung entfernt werden.

Dem erfindungsgemäßen Prüf- und Reinigungsverfahren kann eine Sterilisierung nachgeschaltet sein. Beispielsweise können Verfahren, wie Ethylenoxid-, Formaldehyd-, H₂O₂-Plasma- und Peressigsäuresterilisierung, eingesetzt werden. Dafür wird mindestens ein sterilisierendes Fluid durch die Kanäle und über die Oberflächen der Instrumente geleitet.

Eine Vorrichtung zum Prüfen und Reinigen von Instrumenten für die minimal invasive Chirurgie oder minimal invasive Untersuchung von Körperhöhlen, die in dem erfindungsgemäßen Verfahren eingesetzt werden kann, umfaßt
- einen Prüf- und Reinigungseinsatz für die Instrumente, der Spülkanäle aufweist,
- eine Aufnahmevorrichtung für den Prüf- und Reinigungseinsatz mit Anschlüssen für je einen Spülkanal des Prüf- und Reinigungseinsatzes,
- mindestens ein Fluidreservoir, das über einen Verteiler mit Ventilen und über diese mit den Anschlüssen der Aufnahmevorrichtung verbunden ist,
- eine Pumpe, die zwischen dem Fluidreservoir und dem Verteiler angeordnet ist,
- ein Durchflußprüfgerät, und
- eine Steuereinrichtung, welche mit dem Durchflußprüfgerät sowie dem Verteiler und/oder den Ventilen in Verbindung steht.

Der Prüf- und Reinigungseinsatz für die Instrumente kann in einer bevorzugten Ausführung der Vorrichtung Stirnwände mit Ausnehmungen an deren Oberkanten und unterhalb der Ausnehmungen angeordnete Bohrungen zur Aufnahme der Instrumente, sowie Anschlußstutzen aufweisen. In diese Anschlußstutzen münden in den Stirnwänden verlaufende Kanäle, die aus in einem Sockel des Prüf- und Reinigungseinsatzes verlaufenden Kanälen mit Anschluß an die Anschlüsse der Aufnahmevorrichtung hervorgehen.

Als Durchflußprüfgeräte können beispielsweise Volumenmeßgeräte oder Druckmeßgeräte verwendet werden.

Dabei kann das Durchflußprüfgerät einlaßseitig bezogen auf die Anschlüsse der Aufnahmevorrichtung oder auslaßseitig bezogen auf in der Aufnahmevorrichtung angeordnete Instrumente angeordnet sein.

In der Steuereinrichtung werden die von dem Durchflußprüfgerät übermittelten Informationen mit gerätespezifisch für Standard-Instrumente vorgegebenen Daten verglichen. Wird eine Übereinstimmung der von einer Prüfung eines Instrumentes übermittelten Information mit für einen Instrumententyp gespeicherten Daten festgestellt, wird das betreffende Instrument als ein solches Instrument identifiziert und als durchspülbar erkannt. Daraufhin übermittelt die Steuereinrichtung die geeignete Information an den Verteiler bzw. die Ventile, um eine Reinigung und Desinfektion des Instruments durch gezieltes Zuleiten von Reinigungs- und Desinfektionsmitteln zu bewirken.

Können die vom Durchflußprüfgerät übermittelten Daten in der Steuereinrichtung wegen fehlender Übereinstimmung keinen gerätespezifisch vorgesehenen Standardwerten zugeordnet werden, kann das Gerät nicht identifiziert werden. Das Gerät wird in der Folge als nicht durchspülbar erkannt und zur Aussonderung registriert.

In einer Ausführungsform der Vorrichtung ist die Aufnahmevorrichtung für den Prüf- und Reinigungseinsatz für Instrumente in einer Wanne vorgesehen, von der das Fluid nach Durchströmen der Instrumente aufgefangen wird. In diesem Fall kann das Durchflußprüfgerät hinter einem Ausfluß der Wanne angeordnet sein.

In der Vorrichtung ist auch dafür gesorgt, daß nach erfolgreicher Prüfung neben den Kanälen und Kapillaren auch die äußeren Oberflächen und Gelenke der Instrumente gereinigt werden. Hierzu geeignete Maßnahmen sind bekannt.

In einer Ausführungsform mit Anordnung der Aufnahmevorrichtung in einer Wanne oder einem Behälter können an diesem zusätzlich Ultraschallschwingelemente vorgesehen sein, um hartnäckige Verschmutzungen auf Oberflächen und an Gelenken der Instrumente zu entfernen.

Zusätzlich kann in der Vorrichtung eine Druckstoßeinrichtung vorgesehen sein, die mit dem Durchflußprüfgerät in Verbindung steht und im Falle nicht ausreichenden Durchflusses aktiviert wird. Bei einer Anordnung des Durchflußprüfgerätes hinter dem Ablauf einer Wanne kann zusätzlich ein weiteres, einlaßseitig angeordnetes Durchflußprüfgerät vorgesehen sein, welches zwischen der Druckstoßeinrichtung und dem Verteiler angeordnet ist, um nach einem Druckstoß aufgrund nicht ausreichenden Durchflusses eine zusätzliche genaue Registrierung der Durchflußmenge zu ermöglichen.

An dem mit Prüfungs-, Reinigungs-, Desinfektions- oder Sterilisationsmittel beschickbaren Fluidreservoir kann eine Heizung zur Erwärmung der darin enthaltenen Fluide vorgesehen sein. Durch Öffnen eines am Reservoir vorgesehenen Absperrventils und, wenn gewünscht, zusätzliches Einschalten einer Tankentleerungspumpe kann das Reservoir entleert werden; Prüfungs-, Reinigungs-, Desinfektions- oder Sterilisationsmittel können über vorgesehene Dosierpumpen zudosiert werden.

Bei zusätzlich vorgesehener Sterilisation ist das Sterilisationsfluid vorzugsweise in einem zusätzlichen Reservoir vorgesehen, aus dem es dem Verteiler zugeführt wird. Auch hier ist dafür gesorgt, daß das sterilisierende Fluid auch über die Oberflächen der zu sterilisierenden Instrumente geleitet wird.

Ist die Aufnahmevorrichtung mit dem Prüf- und Reinigungseinsatz in einem Behälter angeordnet, so kann beispielsweise über ein daran angebrachtes Ventil zusätzlich Sterilisationsfluid in den Behälter zur Sterilisierung der Außenflächen der Instrumente eingeführt werden. Nach der Sterilisierung kann über dieses Ventil Sterilisationsfluid aus dem Behälter, gegebenenfalls unter Verwendung einer Pumpe, abgezogen werden.

Zwischen dem bzw. den Fluidreservoir(en) und dem Verteiler kann eine Umwälzpumpe vorgesehen sein. Diese ist beispielsweise bei Verwendung von Wasserstoffperoxid-Plasma nach dem Plasmasterilisationsverfahren auch aus dem Grunde erforderlich, um den Plasmazustand des Wasserstoffperoxids aufrechtzuerhalten.

Zum Erwärmen des Sterilisationsfluids kann am Fluidreservoir eine Heizung vorgesehen sein. Um die Sterilisationstemperatur konstant zu halten, kann darüberhinaus eine zusätzliche Heizung an einem Behälter angebracht sein, in dem die Aufnahmevorrichtung mit dem Prüf- und Reinigungseinsatz angeordnet ist.

Durch eine Öffnung eines am Fluidreservoir vorgesehenen Absperrventils und, wenn erforderlich, zusätzliches Einschalten einer Pumpe kann das Reservoir entleert oder gefüllt werden.

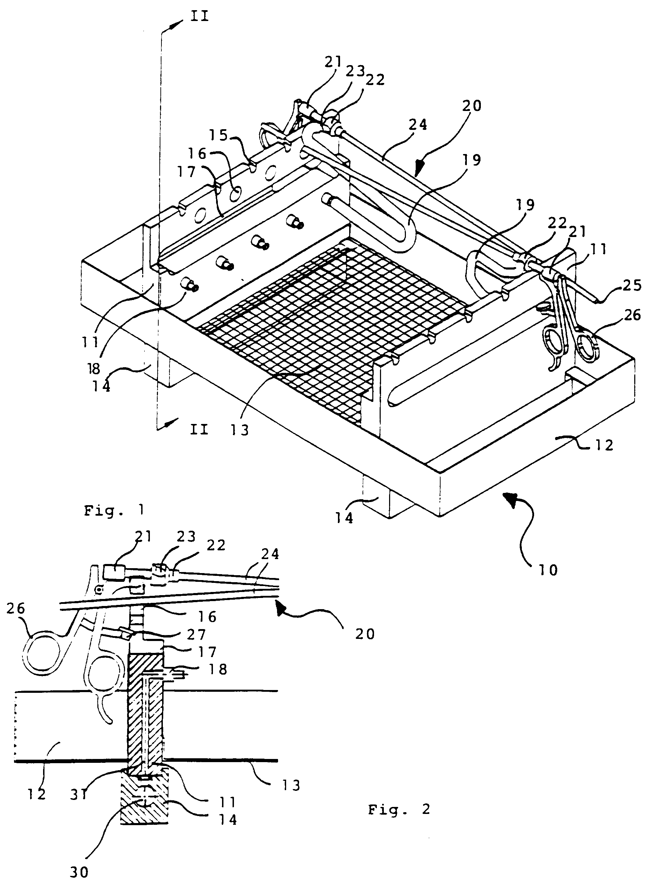

In einer besonders bevorzugten Ausführungsform der Vorrichtung zum Prüfen, Reinigen, Desinfizieren und/oder Sterilisieren von nicht flexiblen MIC-Instrumenten kann der in der Figur 1 gezeigte und im folgenden beschriebene Prüf- und Reinigungseinsatz für MIC-Instrumente verwendet werden, in dem die verschmutzten Instrumente sowohl sämtliche in den erfindungsgemäßen Verfahren beschriebenen Prüf-, Reinigungs- und Desinfektionsschritte als auch die sich u.U. daran anschließende Sterilisation mittels eines Fluids, vorzugsweise eines Plasmas, oder auch in einem Autoklaven durchlaufen und darin gelagert werden können, so daß sie anschließend für einen erneuten Einsatz bei Operationen zur Verfügung stehen.

Sämtliche erfindungsgemäße Prüfungs-, Reinigungs-, Desinfektions- und Sterilisationsschritte können darüberhinaus in einer gemeinsamen Vorrichtung mit den für die im vorhergenden beschriebenen Vorrichtungen beschriebenen Merkmalen ausgeführt werden, wobei für eine Aufbereitung von nicht flexiblen MIC-Instrumenten der Prüf- und Reinigungseinsatz für MIC-Instrumente verwendet werden kann.

Dadurch ist ein direkter Kontakt des Reinigungspersonals mit den verunreinigten Instrumenten während des gesamten Aufarbeitungsverfahrens nur beim Einsetzen der verunreinigten Instrumente in den Prüf- und Reinigungseinsatz erforderlich. Gegebenenfalls müssen die beschickten Einsätze mit den auf Durchlässigkeit geprüften, gereinigten und desinfizierten MIC-Instrumenten nur noch in Sterilisationsvorrichtungen umgesetzt werden, so daß sämtliche Aufarbeitungsschritte minimalen Arbeits- und Personalaufwand erfordern, was eine bedeutende Zeit-und Kostenersparnis mit sich bringt.

Die Erfindung wird im folgenden anhand schematischer Zeichnungen eines Ausführungsbeispiels für nicht flexible MIC-Instrumente näher erläutert; darin zeigen
Fig. 1 eine perspektivische Darstellung eines Prüf- und Reinigungseinsatzes für nicht flexible MIC-Instrumente,
Fig. 2 den Schnitt II-II in Fig. 1,
Fig. 3 ein Schaltschema einer Vorrichtung zum Prüfen und Reinigen von MIC-Instrumenten mit dem Prüf- und Reinigungseinsatz aus Fig. 1 und
Fig. 4 den in einen Waschkorb einer Wasch- und Desinfektionsmaschine eingesetzten Prüf- und Reinigungseinsatz nach Fig. 1.

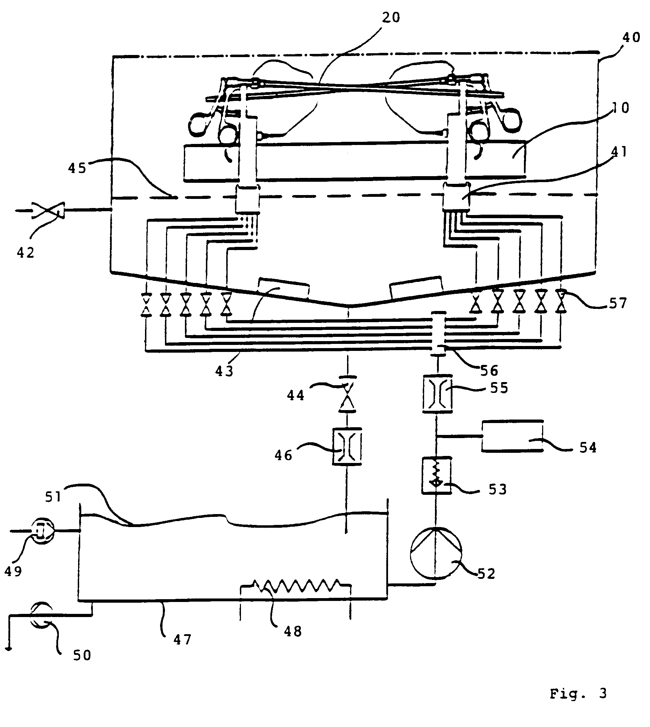

Der in Fig. 1 und Fig. 2 dargestellte Prüf- und Reinigungseinsatz 10 für Vorrichtungen zum Prüfen und Reinigen von nicht flexiblen MIC-Instrumenten 20 hat rechteckige Stirnwände 11, die in axialsymmetrischer Anordnung durch einen horizontalen und an den schmalen Seitenflächen der Stirnwände 11 anliegenden Rahmen 12 sowie ein als Bodenfläche eingesetztes Gitter 13 miteinander verbunden sind. Die Stirnwände 11 sind im oberen Drittel ihrer vertikalen Erstreckung so abgestuft, daß die Dicke der Stirnwände in diesem Bereich nur ein Drittel der im mittleren Bereich gemessenen Dicke beträgt.

Die Stirnwände 11 sind mit ihrer Unterseite in eine mit einer Dichtung versehene Ausnehmung eines Sockels 14 eingesetzt. Jede der Stirnwände 11 hat an ihrer Oberseite halbkreisförmige Ausnehmungen 15 und, weiter unten, eine gleiche Anzahl runder Bohrungen 16, die in bezug auf die Ausnehmungen 15 auf Lücke angeordnet sind. Noch weiter unten hat jede der Stirnwände 11 eine fast ihre gesamte Breite einnehmende, horizontal verlaufende Ausnehmung 17, die zur Hälfte im oberen, verjüngten Stirnwandbereich und zur anderen Hälfte im mittleren Stirnwandbereich liegt.

Die Stirnwände 11 sind miteinander baugleich und sind derart angeordnet, daß die halbkreisförmigen Ausnehmungen 15 einer Stirnwand 11, parallel nach unten verschoben, genau den runden Bohrungen 16 der anderen Stirnwand gegenüberliegen. Unterhalb der Ausnehmung 17 befindet sich im mittleren Bereich der Stirnwände 11 eine der Anzahl der halbkreisförmigen Ausnehmungen 15 und runden Bohrungen 16 gleiche Anzahl nach innen ausgerichteter Anschlußstutzen 18. Die Anschlußstutzen 18 einer Stirnwand sind in bezug auf die runden Bohrungen 16 derselben Stirnwand auf Lücke angeordnet. Die Anschlußstutzen 18 sind für den Anschluß von Druckschläuchen 19 vorgesehen.

Die MIC-Instrumente 20 werden in den Prüf- und Reinigungseinsatz 10 zwischen je einem Gelenk 21 und einem Verbindungsstück 22 mit Durchspülstutzen 23 in den halbkreisförmigen Ausnehmungen 15 der Stirnwände 11 so aufgenommen, daß ein an das Verbindungsstück 22 anschließendes stabförmiges Bauteil 24 mit durchgehendem Kanal 25 mit seinem offenen, distalen Ende durch die auf der anderen Seite vorgesehene, gegenüberliegende runde Bohrung 16 geführt wird und daß das MIC-Instrument 20 in Richtung des offenen Endes seines stabförmigen Bauteils 24 nach unten geneigt ist. Das offene, distale Ende des stabförmigen Bauteils 24 befindet sich in dieser Anordnung über dem Zwischenraum zwischen der nach außen weisenden Fläche der Stirnwand 11 und dem ihr gegenüberliegenden Abschnitt des Rahmens 12.

Ein zweiter Satz MIC-Instrumente 20 kann in gleicher Weise, jedoch ausgehend von der jeweils gegenüberliegenden Stirnwand 11 parallel zu den erstgenannten MIC-Instrumenten angeordnet werden, jedoch gegen diese seitlich versetzt. Ein an einem Griff 26 angebrachtes Bauelement 27 befindet sich in dieser Anordnung mit seinem distalen Ende in der horizontal verlaufenden Ausnehmung 17 des Prüf- und Reinigungseinsatzes 10. Der am Verbindungsstück 22 des MIC-Instruments 20 vorgesehene Durchspülstutzen 23 kann mit dem freien Ende des Druckschlauchs 19 verbunden werden, sodaß der durch das stabförmige Bauteil 24 durchgehende Kanal 25 durchspült werden kann.

Fig. 2 zeigt die Anordnung der fluidführenden Kanäle im Prüf- und Reinigungseinsatz 10, die in die Anschlußstutzen 18 münden. Fluid wird von einer unterhalb des Prüf- und Reinigungseinsatzes 10 angeordneten, nicht abgebildeten Fluidquelle durch horizontale Kanäle 30 im Sockel 14 der stirnwände 11 geführt, aus denen das Fluid über senkrecht dazu verlaufende, in den Stirnwänden 11 nach oben führende Kanäle 31 den Anschlußstutzen 18 zugeführt wird.

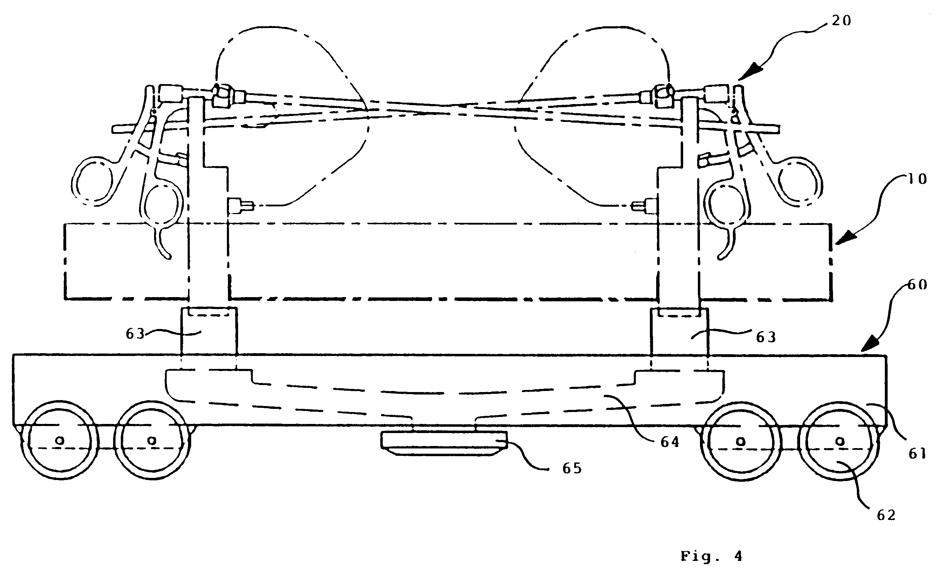

Die in Fig. 3 dargestellte Vorrichtung zum Prüfen und Reinigen von MIC-Instrumenten weist eine Edelstahlwanne 40 auf, die eine Aufnahmevorrichtung 41 aufnimmt. Diese ist in Form eines Anschlußblocks für den mit MIC-Instrumenten 20 beschickten Prüf- und Reinigungseinsatz 10 ausgeführt und weist Anschlüsse für je einen Spülkanal 30 auf. Zum Reinigen der Instrumente 20 kann die Wanne 40 über ein Ventil 42 mit Flüssigkeit gefüllt werden, so daß die Aufnahmevorrichtung 41 mit eingesetztem Prüf- und Reinigungseinsatz 10 vollständig mit Flüssigkeit bedeckt ist. Die Wanne 40 ist darüberhinaus mit Ultraschallschwingelementen 43 ausgestattet. Durch Fluten der Wanne 40 und Einschalten der Ultraschallschwingelemente 43 können schwierig zu reinigende Oberflächenbereiche der MIC-Instrumente zusätzlich gereinigt werden. Die Wanne 40 besitzt einen Ablauf, der durch ein Absperrventil 44 gesteuert werden kann, und einen Niveauregler 45, der die Füllhöhe der Wanne kontrolliert.

Hinter dem Absperrventil 44 der Wanne 40 ist ein Durchflußprüfgerät 46 angeordnet, das im Falle einer speziellen Ausführungsform der Prüfung der Instrumente 20 nach Öffnung des Absperrventils 44 das Volumen der hindurchfließenden Flüssigkeit mißt, die nach definiertem Durchströmen der Kanäle 25 der MIC-Instrumente 20 von der Wanne 40 aufgefangen wird.

Die durch das Durchflußprüfgerät 46 fließende Flüssigkeit wird von einem Reservoir 47 aufgenommen. Das Reservoir 47 ist mit einer Heizung 48 ausgestattet, und über eine Dosierpumpe 49 kann Reinigungs- und Desinfektionsmittel zudosiert werden. Zum Entleeren ist eine Ablaufpumpe 50 vorgesehen. Die Füllhöhe des Reservoirs 47 wird durch einen Niveauregler 51 kontrolliert. An das Reservoir 47 ist ferner eine Umwälzpumpe 52 angeschlossen, hinter der druckseitig ein Rückschlagventil 53 angeordnet ist. Hinter diesem befindet sich eine Druckstoßeinrichtung 54 und ein weiteres Durchflußprüfgerät 55. Das Reinigungs- und Desinfektionsmittel wird über einen daran anschließend vorgesehenen Verteiler 56 Ventilen 57 zugeleitet, die über die Anschlüsse der Aufnahmevorrichtung 41 die Fluidzuleitung zu einzelnen Spülkanälen 30,31 im Prüf- und Reinigungseinsatz 10 steuern.

In einer nicht abgebildeten Steuereinrichtung, die in Kontakt mit einem oder beiden Durchflußprüfgerät(en) 46,55 sowie dem Verteiler 56 und/oder den Ventilen 57 steht, wird die von dem bzw. den Durchflußprüfgerät(en) 46,55 übermittelten Information mit gerätespezifisch für Standard-MIC-Instrumente 20 vorgegebenen Daten verglichen. Wird eine Übereinstimmung der von einer Prüfung eines MIC-Instrumentes 20 übermittelten Information mit für einen MIC-Instrumententyp gespeicherten Daten festgestellt, wird das betreffende Instrument 20 als ein solches Instrument identifiziert und als durchspülbar erkannt. Daraufhin übermittelt die Steuereinrichtung die geeignete Information an den Verteiler 56 bzw. die Ventile 57, um eine Reinigung und Desinfektion des MIC-Instruments 20 durch Zuleiten von Reinigungs- und Desinfektionsmitteln zu bewirken.

Können die von dem bzw. den Durchflußprüfgerät(en) 46,55 übermittelten Daten in der Steuereinrichtung keinen gerätespezifisch vorgesehenen standardwerten aufgrund fehlender Übereinstimmung zugeordnet werden, kann das MIC-Instrument 20 nicht identifiziert werden. Das Instrument 20 wird in der Folge als nicht durchspülbar erkannt und zur Aussonderung registriert.

Alternativ kann die Wanne oder ein andersartiger Behälter 40 über ein Ventil 42 mit Sterilisationsfluid, gegebenenfalls unter Druck, gefüllt werden, so daß die Aufnahmevorrichtung 41 mit eingesetztem Prüf- und Reinigungseinsatz 10 vollständig von Sterilisationsfluid umströmt ist. Der Behälter 40 besitzt einen Ablauf, der über ein Absperrventil 44 gesteuert werden kann, und der mit einem Reservoir 47 für Sterilisationsfluid verbunden ist. Das Fluidreservoir 47 ist mit einer Heizung 48 ausgestattet und kann mittels einer Pumpe 50 gefüllt oder entleert werden. An das Fluidreservoir 47 ist ferner eine Umwälzpumpe 52 angeschlossen, hinter der druckseitig ein Rückschlagventil 53 angeordnet ist. Sterilisationsfluid wird über einen daran anschließend vorgesehenen Verteiler 56 Ventilen 57 zugeleitet, die über die Anschlüsse der Aufnahmevorrichtung 41 die Fluidzuleitung zu einzelnen Spülkanälen 30,31 im Prüf- und Reinigungseinsatz 10 steuern.

Die Bauteile mit den Bezugszeichen 43, 45, 46, 49, 51, 54, 55 sind in der Vorrichtung zum Sterilisieren von MIC-Instrumenten 20 nicht erforderlich.

Fig. 4 ist eine schematische Darstellung des in einen Waschkorb 60 einer nicht abgebildeten Wasch- und Desinfektionsmaschine eingesetzten Prüf- und Reinigungseinsatzes 10 aus Fig. 1. Der Waschkorb 60 umfaßt eine flache Wanne 61, die auf Rollen 62 geführt ist, wodurch das Einschieben in Wasch- und Desinfektionsmaschinen erleichtert wird. Der Prüf- und Reinigungseinsatz 10 wird über die Bodenflächen der Sockel 14 der Stirnwände 11 von Einsätzen 63 in der Wanne 61 des Waschkorbs 60 aufgenommen, wobei beide Einsätze 63 ihrerseits von einem Verbindungselement 64 aufgenommen werden, welches an der Unterseite der Wanne 61 verläuft und mittig mit einem Anschluß 65 versehen ist.

Fluid kann aus einer nicht abgebildeten Fluidquelle, gegebenenfalls über einen Verteiler und nachgeschaltete Ventile, zu dem Anschluß 65 des Verbindungselements 64 geführt werden. Von dem Anschluß 65 ausgehend, strömt das Fluid dann durch einen oder mehrere freigegebene, in dem Verbindungselement 64 vorgesehene Kanäle zu den Einsätzen 63 und von dort zu jeweils in den Sockeln 14 des Prüf- und Reinigungseinsatzes 10 verlaufenden Kanälen 30. Nachfolgend kann das Fluid die daran anschließenden Kanäle 31 und, daraufhin, die durch die stabförmigen Bauteile 24 durchgehenden Kanäle 25 der MIC-Instrumente 20 durchspülen.

Durch Anpassen des Prüf- und Reinigungseinsatzes für eine Verwendung für Endoskope, indem Anschlußstutzen für eine vollständige Durchströmung sämtlicher Kanäle und Kapillaren vorgesehen werden, können die Vorrichtungen jedoch ohne weitere Änderungen für eine Prüfung, Reinigung, Desinfektion und/oder Sterilisation von Endoskopen verwendet werden.

## Patentansprüche

1. Verfahren zum Prüfen und Reinigen von einem oder mehreren Instrument(en) (20) für die minimal invasive Chirurgie oder minimal invasive Untersuchung von Körperhöhlen mit mindestens je einem durchgehenden Kanal (25), in dem
(a) der Kanal (25) des bzw. jedes Instruments (20) an eine Fluidquelle (47) angeschlossen wird;
(b) der Kanal (25) auf Durchlässigkeit geprüft wird, indem ihm Fluid aus der Fluidquelle (47) zugeführt wird;
(c) bei zu geringer oder fehlender Durchströmung das betreffende Instrument (20) als nicht ausreichend durchspülbar erkannt und zur Aussonderung registriert wird und
(d) bei ausreichender Durchströmung das betreffende Instrument (20) als durchspülbar erkannt, und mit Desinfektions- und/oder Reinigungsmitteln nachgespült wird,
wobei
(e) bei der Durchlässigkeitsprüfung eine Menge des in einer Zeiteinheit den Kanal (25) durchströmenden Fluids bestimmt wird
und
(f) das bestimmte Fluidvolumen mit gerätespezifisch vorgegebenen Daten verglichen wird,
dadurch gekennzeichnet, daß
(g) anhand des Ergebnisses der Durchlässigkeitsprüfung das bzw. die Instrument(e) (20) hinsichtlich seines bzw. ihres Gerätetyps identifiziert werden und
(h) abhängig davon Bedingungen für das Nachspülen mit Desinfektions- und/oder Reinigungsmitteln festgelegt werden.

2. Verfahren nach Anspruch 1,
dadurch gekennzeichnet,
daß die Kanäle (25) einer Mehrzahl von Instrumenten (20) für jedes Instrument (20) einzeln nacheinander auf Durchlässigkeit geprüft werden, indem nur jeweils dem Kanal (25) eines einzelnen Instruments (20) Fluid aus der Fluidquelle (47) zugeführt wird.

3. Verfahren nach Anspruch 1,
dadurch gekennzeichnet,
daß die Kanäle (25) einer Mehrzahl von Instrumenten (20) gemeinsam auf Durchlässigkeit geprüft werden, indem den Kanälen (25) gemeinsam Fluid aus der Fluidquelle (47) zugeführt wird.

4. Verfahren nach einem der Ansprüche 1 bis 3,
dadurch gekennzeichnet, daß
die Menge des in einer Zeiteinheit den Kanal (25) durchströmenden Fluids bestimmt wird, indem ein Volumen des in der Zeiteinheit den Kanal (25) durchströmenden Fluids gemessen wird.

5. Verfahren nach einem der Ansprüche 1 bis 3,
dadurch gekennzeichnet, daß
die Menge des in einer Zeiteinheit den Kanal (25) durchströmenden Fluids bestimmt wird, indem der Kanal (25) durch Zuführen von Fluid aus der Fluidquelle (47) unter Druck gesetzt wird, ein Druckabfall mit der Zeit infolge eines Fluiddurchstroms durch den Kanal (25) gemessen und der Druckabfall mit der Zeit ins Verhältnis zu der Menge des in einer Zeiteinheit den Kanal (25) durchströmenden Fluids gesetzt wird.

6. Verfahren nach einem der Ansprüche 1 bis 5,
dadurch gekennzeichnet, daß
bei nicht ausreichender Durchströmbarkeit eines oder mehrerer Instrumente (20) automatisch Maßnahmen ergriffen werden, um zu einer vollständigen Durchströmbarkeit zu gelangen.

7. Verfahren nach Anspruch 6,
dadurch gekennzeichnet, daß
als Maßnahme, um zu einer vollständigen Durchströmbarkeit zu gelangen, ein Druckstoß eingesetzt wird.

8. Verfahren nach einem der Ansprüche 1 bis 7,
dadurch gekennzeichnet, daß
in Abhängigkeit von dem Ergebnis der Durchlässigkeitsprüfung die Instrumente (20) einzeln oder in Gruppen mit Desinfektions- und/oder Reinigungsmitteln nachgespült werden.

9. Verfahren nach einem der Ansprüche 1 bis 8,
dadurch gekennzeichnet, daß
festsitzende Verschmutzungen der Instrumente (20) zusätzlich durch Ultraschall abgelöst werden.

10. Verfahren nach einem der Ansprüche 1 bis 9,
dadurch gekennzeichnet, daß
die Instrumente (20) nach dem Prüfen und Reinigen sterilisiert werden, indem mindestens ein sterilisierendes Fluid durch die Kanäle (25) der Instrumente (20) und über deren Oberflächen geleitet wird.

11. Verfahren nach Anspruch 10,
dadurch gekennzeichnet, daß
als sterilisierendes Fluid Ethylenoxid, Formaldehyd, H₂O₂-Plasma, Peressigsäure oder ein anderes Fluid verwendet wird.

## Claims

1. Method for the testing and cleaning of one or more instrument(s) (20) for minimal invasive surgery or minimal invasive examination of body cavities, with in each case at least one continuous channel (25), in which method
(a) the channel (25) of the or each instrument (20) is connected to a fluid source (47);
(b) the channel (25) is tested for penetrability by means of fluid being conveyed to it from the fluid source (47);
(c) if there is too little or no throughflow, the instrument (20) in question is recognized as being one that cannot be adequately flushed and is registered for rejection, and
(d) if the throughflow is sufficient, the instrument (20) in question is recognized as being one that can be flushed and it is re-flushed with disinfecting and/or cleaning agents, wherein
(e) a quantity of the fluid flowing through the channel (25) in a unit of time is determined in the penetrability test, and
(f) the fluid volume determined is compared with preset data specific to the device,
characterized in that
(g) on the basis of the result of the penetrability test, the instrument or instruments (20) is/are identified in terms of its/their device type, and
(h) as a function of this, conditions are established for the re-flushing with disinfecting and/or cleaning agents.

2. The method as claimed in claim 1,
characterized in that the channels (25) of a plurality of instruments (20) are tested individually one after the other for penetrability for each instrument (20), by means of fluid from the fluid source (47) being conveyed only to the respective channel (25) of one individual instrument (20).

3. The method as claimed in claim 1,
characterized in that the channels (25) of a plurality of instruments (20) are tested jointly for penetrability, by means of fluid from the fluid source (47) being conveyed jointly to the channels (25).

4. The method as claimed in one of claims 1 to 3,
characterized in that the quantity of the fluid flowing through the channel (25) in a unit of time is determined by measuring a volume of the fluid flowing through the channel (25) in the unit of time.

5. The method as claimed in one of claims 1 to 3,
characterized in that the quantity of the fluid flowing through the channel (25) in a unit of time is determined by means of the channel (25) being pressurized by admission of fluid from the fluid source (47), a pressure drop over time as a result of a flow of fluid through the channel (25) being measured, and the pressure drop over time being set in proportion to the quantity of the fluid flowing through the channel (25) in a unit of time.

6. The method as claimed in one of claims 1 to 5,
characterized in that in the event of one or more instruments (20) having insufficient throughflow, measures are automatically taken in order to achieve complete throughflow.

7. The method as claimed in claim 6,
characterized in that the measure for achieving complete throughflow involves implementing a pressure surge.

8. The method as claimed in one of claims 1 to 7,
characterized in that as a function of the result of the penetrability test, the instruments (20) are re-flushed individually or in groups with disinfecting and/or cleaning agents.

9. The method as claimed in one of claims 1 to 8,
characterized in that obstinate contamination of the instruments (20) is additionally detached by ultrasound.

10. The method as claimed in one of claims 1 to 9,
characterized in that the instruments (20) are sterilized after having been tested and cleaned by means of at least one sterilizing fluid being conveyed through the channels (25) of the instruments (20) and over the surface thereof.

11. The method as claimed in claim 10,
characterized in that the sterilizing fluid used is ethylene oxide, formaldehyde, H₂0₂ plasma, peracetic acid or another fluid.

## Revendications

1. Procédé d'examen et de nettoyage d'un instrument ou de plusieurs instruments (20) pour la chirurgie invasive minimale ou pour l'examen invasif minimal de cavités du corps, comportant au moins chacun un canal ininterrompu (25), dans le cas duquel
(a) le canal (25) de l'instrument ou de chaque instrument (20) est relié à une source de fluide (47) ;
(b) le canal (25) est examiné au point de vue liberté de passage en y amenant du fluide en provenance de la source de fluide (47) ;
(c) en cas d'écoulement trop faible ou inexistant, l'instrument en question (20) est reconnu comme ne pouvant pas être suffisamment nettoyé et il est porté sur une liste de retrait et
(d) en cas d'écoulement suffisant l'instrument (20) est reconnu comme pouvant être nettoyé et il est ensuite nettoyé avec des agents de désinfection et/ou de nettoyage,
dans le cas duquel
(e) lors de l'examen de liberté de passage, est déterminée une quantité du fluide qui doit passer dans le canal (25) dans une unité de temps et
(f) le volume de fluide déterminé est comparé avec des données prescrites, spécifiques, de l'appareil,
caractérisé par le fait que
(g) à l'aide du résultat de l'examen de liberté de passage, l'instrument ou les instruments (20) sont identifiés du point de vue de leur type d'appareil et
(h) en fonction de cela, des conditions sont déterminées pour le lavage, qui suit, avec des agents de désinfection et/ou de nettoyage.

2. Procédé selon la revendication 1,
caractérisé
par le fait que les canaux (25) d'une pluralité d'instruments (20) sont examinés au point de vue liberté de passage pour chaque instrument (20) isolément l'un après l'autre, en ce sens que du fluide en provenance de la source du fluide (47) n'est amené chaque fois qu'au canal (25) d'un unique instrument (20).

3. Procédé selon la revendication 1,
caractérisé
par le fait que les canaux (25) d'une pluralité d'instruments (20) sont examinés en commun au point de vue liberté de passage en ce sens que du fluide en provenance de la source de fluide (47) est amené aux canaux (25) en commun.

4. Procédé selon l'une des revendications 1 à 3,
caractérisé
par le fait que la quantité du fluide qui doit passer dans le canal (25) dans une unité de temps est déterminé en ce sens qu'est mesuré un volume du fluide qui doit passer dans le canal (25) dans l'unité de temps.

5. Procédé selon l'une des revendications 1 à 3,
caractérisé
par le fait que la quantité du fluide qui doit passer dans le canal (25) dans une unité de temps est déterminée en ce sens que le canal (25) est mis sous pression par amenée de fluide en provenance de la source de fluide (47), qu'est mesurée une chute de pression en fonction du temps par suite d'un passage du fluide dans le canal (25) et qu'est établie la chute de pression en fonction du temps en relation avec la quantité du fluide qui doit passer dans le canal (25) en une unité de temps.

6. Procédé selon l'une des revendications 1 à 5,
caractérisé
par le fait qu'en cas de capacité d'écoulement insuffisante d'un ou de plusieurs instruments (20) des mesures sont automatiquement prises pour parvenir à une complète capacité d'écoulement.

7. Procédé selon la revendication 6,
caractérisé
par le fait que comme mesure pour parvenir à une complète capacité d'écoulement est employé un coup de bélier.

8. Procédé selon l'une des revendications 1 à 7, caractérisé
caractérisé
par le fait qu'en fonction du résultat de l'examen de liberté de passage, les instruments (20) sont ensuite lavés, isolément ou en groupes, avec des agents de désinfection et/ou de nettoyage.

9. Procédé selon l'une des revendications 1 à 8,
caractérisé
par le fait que des saletés bloquées dans les instruments (20) sont en outre désagrégées par ultrasons.

10. Procédé selon l'une des revendications 1 à 9,
caractérisé
par le fait qu'après examen et nettoyage les instruments (20) sont stérilisés en ce sens qu'au moins un fluide de stérilisation est envoyé dans les canaux (25) des instruments (20) et sur leurs surfaces.

11. Procédé selon la revendication 10,
caractérisé
par le fait que comme fluide de stérilisation est employé de l'oxyde d'éthylène, du formaldéhyde, du plasma H₂O₂, de l'acide peracétique ou un autre fluide.
